# EUROPEAN PATENT SPECIFICATION

(11) **EP 3 146 973 B1**
(45) Date of publication and mention of the grant of the patent: **06.05.2020**
(21) Application number: 15819530.5
(22) Date of filing: 02.03.2015
(51) Int. Cl.: A61K 8/98, A61P 17/14, A61K 35/28, A61Q 7/00, C12N 5/0775, A61K 35/51

(54) **HAIR GROWTH-PROMOTING FUNCTION OF SMALL-SIZED STEM CELLS AND USE THEREOF**
HAARWACHSTUMSFÖRDERNDE FUNKTION VON KLEINEN STAMMZELLEN UND VERWENDUNG DAVON
FONCTION FAVORISANT LA REPOUSSE DES POILS DES CELLULES SOUCHES DE PETITES TAILLES ET SON UTILISATION

(30) Priority: 07.07.2014 US 201462021362 P; 19.09.2014 US 201462052635 P
(43) Date of publication of application: 29.03.2017
(73) Proprietor: Medipost Co., Ltd., Bundang-gu, Seongnam-si Gyeonggi-do 13494 (KR)
(72) Inventor: YANG, Yoon Sun, Gyeonggi-do 13494 (KR); OH, Wonil, Gyeonggi-do 13494 (KR); LEE, Jang Young, Gyeonggi-do 13494 (KR); CHOI, Soo Jin, Gyeonggi-do 13494 (KR); JEON, Hong Bae, Gyeonggi-do 13494 (KR); KIM, Ju-Yeon, Gyeonggi-do 13494 (KR); LIM, Hoon, Gyeonggi-do 13494 (KR)
(74) Representative: Prüfer & Partner mbB Patentanwälte · Rechtsanwälte
(86) International application number: PCT/KR2015/002007
(87) International publication number: WO 2016/006788

(56) References cited:
- WO-A1-2009/061024
- WO-A1-2013/067038
- WO-A2-2007/100845
- WO-A2-2010/107287
- WO-A2-2012/047733
- WO-A2-2012/091321
- US-A1- 2002 168 765
- US-A1- 2005 048 034
- US-A1- 2005 048 034
- US-A1- 2006 057 126
- US-A1- 2012 251 504
- US-A1- 2014 161 774
- US-A1- 2015 037 435
- LIANG DONG ET AL: "Treatment of MSCs with Wnt1a-conditioned medium activates DP cells and promotes hair follicle regrowth", SCIENTIFIC REPORTS, vol. 4, 25 June 2014 (2014-06-25), XP055250810, DOI: 10.1038/srep05432
- YOO, B. Y.: "Application of mesenchymal stem cells derived from bone marrow and umbilical cord in human hair multiplication", Journal of dermatological science, 2010, pages 74-83, XP027437274, DOI: doi:10.1016/j.jdermsci.2010.08.017
- YOO, B. Y.: Evaluation of Human Umbilical Cord-derived Mesenchymal Stem Cells on In Vivo Hair Inducing Activity, 2009, page 179,

## Description

### BACKGROUND

### 1. Field of the Invention

The present invention relates to a composition for preventing hair loss and stimulating hair growth, which contains small-sized stem cells, particularly, small-sized stem cells having a diameter of 8 µm or less, or a conditioned medium thereof as an active ingredient, a method of preparing the same, and a use of the same. The present invention relates to a use of the composition utilizing a hair growth stimulating function of the small-sized stem cells having a diameter of 8 µm or less to considerably increase activity of hair follicle stem cells in a telogen phase.

### 2. Discussion of Related Art

According to the industrial development, environmental pollution, stress and the advance of aging, alopecia or hair thinning has gradually developed, and interests in the life quality and appearance are increasing in well-being age.

Alopecia which is the loss of hair from a scalp occurs due to various causes, for example, internal causes such as genetic constitution or the action of male hormone; mental stress in everyday lives; and external causes such as the accumulation of lipoperoxide in a scalp. It is known that symptoms of hair loss are caused by very complicated processes.

Baldness does not mean the lack of hair by falling, but gradually thinning of hair and becoming of finer and softer hair, and according to the progression of baldness, a dermal papilla in a hair root is smaller. As the dermal papilla is smaller, hair is thinner, a hair cycle is shorter, and newly-growing hair becomes further thinner. When the baldness is continuously progressing, the hair is softer, the hair cycle is further shorter, and the hair is grown a bit and then fallen. In addition, alopecia areata known to be caused by an autoimmune disease, and temporal hair loss occurring after physical or mental stress such as endocrinopathy, nutrition deficiency, drugs or delivery has also received an attention.

Recently, hair loss including male pattern hair loss and female obese hair loss is gradually expanding in a younger generation. According to the reference issued by Korean Institute for Health and Welfare Policy of National Health Insurance Corporation in 2009, the number of domestic hair loss patients in 2008 increased by 60% or more, compared with that in 2001, and the number of children and adolescent hair loss patients has increased by approximately 6.4% for five years from 21,643 in 2006 to 23,025 in 2011.

To improve such hair loss, various types of hair growth solutions are commercially available. Today, according to the research of Economy21, a Korean hair care service market scale is 80% for cosmetics and sanitary aids, and 20% for medications, and among this, the number of hair loss patients coming to a hospital is only 5%. Today, dissatisfied ones of the users of hair care products are 72.7%. However, there are only two drugs approved by the U.S. Food and Drug Administration (FDA) such as minoxidil and finasteride released in 1998 approximately 15 years ago, and thus various causes of alopecia cannot be completely recovered with only two drugs. Particularly, Propecia® made of Finasteride® is an anti hair loss preparation, but not a hair growth solution. Propecia® blocks a 5-α reductase to inhibit DHT production, and delays a male pattern hair loss as long as possible.

Since the hair growth solutions on the market (e.g., Minoxidil® and Finasteride®) have a side effect caused by the application of a hormone drug and are effective only in a part in which a hair root is activated, they are effective in prevention of hair loss, but insignificantly effective in the growth of hair in a telogen phase for a long time, and have to be continuously taken or applied. Therefore, for alopecia or thinning hair, the development of economical and stable technology are needed. In Korea, compared to the developed countries, the development of studying a medicine for alopecia is technically deficient, and the study and development relating to the prevention of hair loss, the stimulation of hair growth, and regeneration of hair are very urgent issues. While, as the study relating to hair loss, literatures relating to regeneration of hair and the hair root have been reported since 1950, due to the lack of reproducibility, the hair regeneration has been considered impossible for last 50 years.

However, in 1990, hair follicle stem cells were first found (Cell, 1990), human-derived hair follicle stem cells were first isolated (J. Clin. Invest, 2006), and a result for possibility to regenerate hair follicles which was impossible to be realized was reported (Nature, 2007) by Professor Costarelis of the School of Medicine at University of Pennsylvania, and thus possibility of studying a fundamental cure of baldness was open.

Recently, a method of treating alopecia using a gene and a method of treating alopecia using a stem cell are being developed. As a background technology of the present invention, in Korean Patent No. 10-0771171 (Oct. 29, 2007), a method of isolating and proliferating hair follicle stem cells, and differentiating the stem cells into hair follicle cells, and a composition for treating hair loss are disclosed, and in Korean Patent Publication No. 10-2008-0097593 (Nov. 6, 2008), a cell therapy product manufactured by suitably mixing adipose-derived stem cells and hair follicle cells is disclosed. In addition, in Korean Patent No. 10-1218101 (Jan. 3, 2013), a composition for stimulating hair growth or preventing hair loss including a conditioned medium of fetal mesenchymal stem cells in an amniotic fluid as an active ingredient is disclosed.

However, a composition for stimulating hair growth or preventing hair loss exhibiting an excellent effect in consideration of specific efficacy of small-sized stem cells has not been reported.

Accordingly, the inventors confirmed that various sizes of cells are generally collected when mesenchymal stem cells are cultured, and found that stem cells having a specific size of diameter or less exhibited a very excellent effect of stimulating hair growth by comparing capability of stimulating hair growth caused by the activation of hair follicle stem cells according to sizes of mesenchymal stem cells derived from various tissues based on the possibility of effects according to a cell size, and thus the present invention was completed.

### [Prior Art Documents]

### [Patent Documents]

1.Korean Patent No. 10-0771171 (Oct. 29, 2007)
2. Korean Patent No. 10-1422559 (Jul. 17, 2014)
3. Korean Patent Publication No. 10-2013-0009117 (Jan. 23, 2013)
4. Korean Patent Publication No. 10-2014-0125735 (Oct. 29, 2014)
5. Korean Patent Publication No. 10-2008-0097593 (Nov. 6, 2008)
6. Korean Patent No. 10-1218101 (Jan. 3, 2013)
7. WO 2012/091321

WO 2012/091321 discloses a method of promoting hair growth, which involves cultivating adipose stem cells and using the culture liquid. The cell size is not restricted to the size as defined in the present application.

### [Non-patent Documents]

1. BY Yoo, YK Seo, JK Park, et al., Effect of Mesenchymal Cells on Human Hair Growth and Death, Korean J. Chem. Eng., 25(2), 295 (2008).
2. VA Botchkarev, J Kishimoto, Molecular control of epithelial-mesenchymal interactions during hair follicle cycling, J. Invest Dermatol. Symp. Proc., 8(1), 46 (2003).
3. D Fiszer, M Kurpisz, T Siminiak, Stem cell therapy as the reinforcement of organ regeneration, Artif. Organs., 29(5),366 (2005).
4. H Oshima, A Rochat, C Kedzia, et al., Y Barrandon, Morphogenesis and renewal of hair follicles from adult multipotent stem cells, Cell, 104(2), 233 (2001).
5. KS Stenn, G Cotsarelis, Bioengineering the hair follicle: fringe benefits of stem cell technology, Curr Opin.Biotechnol., 16(5), 493 (2005).
6. S Tiede, JE Kloepper, E Bodo, et al., Hair follicle stem cells: Walking the maze, Eur. J. Cell Biol., 86(7), 355 (2007).
7. K Kataoka, RJ Medina, T Kageyama, et al., Participation of adult mouse bone marrow cells in reconstitution of skin, Am. J. Pathol., 163(4), 1227 (2003).
8. C Blanpain, WE Lowry, A Geoghegan, et al., Self-renewal, multipotency and the existence of two cell populations within an epithelial stem cell niche, Cell, 118(5), 635 (2004).
9. MJ Hoogduijn, E Gorjup, PG Genever, Comparative characterization of hair follicle dermal stem cells and bone marrow mesenchymal stem cells, Stem Cells Dev., 15(1), 49 (2006).

Throughout the specification, various theses and patents are referred and citations thereof are represented.

### DETAILED DESCRIPTION OF THE INVENTION

### Technical Problem

The present invention is to use a size of a stem cell having a considerably excellent hair growth stimulating function, which stimulates an activity of hair follicle stem cells, and is directed to providing a composition for preventing hair loss and stimulating hair growth, which contains small-sized stem cells having a diameter of 8 µm or less or, a conditioned medium thereof as an active ingredient.

The present invention is also directed to providing a method of preventing hair loss and stimulating hair growth using the composition. The invention is defined by the appended claims.

### Technical Solution

To solve the object, the present invention provides a function of stem cells having a small-sized diameter to stimulate hair growth and various uses thereof.

As an exemplary embodiment of the present invention, a composition for use in therapy of preventing hair loss and stimulating hair growth, which contains small-sized stem cells having a diameter of 8 µm or less, or a conditioned medium thereof as an active ingredient, is provided.

The effect of preventing hair loss and stimulating hair growth is obtained because the small-sized stem cells having a diameter of 8 µm or less, or a conditioned medium thereof has a function of activating hair follicle stem cells, and more particularly, has effects of (i) reduced time for converting a telogen phase into an anagen phase in a hair cycle, (ii) normalization of hair cycle regulation, and (iii) an increase in generation of dermal papilla cells.

Here, the small-sized stem cells having a diameter of 8 µm or less may be at least one type of stem cells selected from the group consisting of bone marrow-, umbilical cord blood-, adipose-, blood-, liver and intestine-, skin-, gastrointestinal tract-, placenta-, nerve-, adrenal-, epithelium- and uterus-derived human adult stem cells, and embryonic stem cells, and preferably, bone marrow-, umbilical cord blood- or adipose-derived, and more preferably, umbilical cord blood-derived adult stem cells, and most preferably, umbilical cord blood-derived mesenchymal stem cells. Further, human-derived umbilical cord blood is most preferably used.

In addition, the conditioned medium may be any medium suitable for the growth of animal cells as a basal medium, and as an unlimited example, a minimal essential medium (MEM), a Dulbecco modified Eagle medium (DMEM), a Roswell Park Memorial Institute medium (RPMI), or a keratinocyte-serum free medium (K-SFM) may be used. Preferably, an α-MEM medium is used.

The composition may be provided by being prepared in a form of a pharmaceutical composition or a cosmetic composition.

Meanwhile, in another exemplary embodiment of the present invention, a method of using the composition for preventing hair loss and stimulating hair growth, which contains small-sized stem cells having a diameter of 8 µm or less or a conditioned medium thereof as an active ingredient, through percutaneous administration using injection, is provided.

In another exemplary embodiment of the present invention, a non-therapeutic, cosmetic use of a composition for preventing hair loss and stimulating hair growth, wherein the composition is comprising small-sized stem cells or a conditioned medium thereof as defined herein, is provided.

Also described herein is a method of treating a hair loss using small-sized stem cells having a diameter of 8 µm or less or a conditioned medium thereof as an active ingredient, is provided, too.

In those method, most preferably, the stem cells or composition thereof are administered through percutaneous administration using injection. Here, the injection is effectively performed by the way that the composition is administrated into the dermis of a target by placing a hole of a syringe needle upward

Accordingly, the present invention is completed based on the finding that the small-sized stem cells having a diameter of 8 µm or less, or a conditioned medium thereof has the most excellent capability to the effect of preventing hair loss and stimulating hair growth compared to conventional heterogeneous cells in which various sizes of stem cells are mixed, and provides an excellent effect of small-sized stem cells having a diameter of 8 µm or less to prevent hair loss and stimulate hair growth according to the activation of hair follicle stem cells, and various uses thereof.

Exemplary embodiments of the present invention will be described in detail below with reference to the accompanying drawings.

The terminology used herein is defined as follows.

The "stimulation of hair growth" or "prevention of hair loss" has a similar meaning, and includes another term used in the art, such as stimulation of hair raising or promoting hair growth promotion and preventing hair weakening or hair loss.

The "stem cell" means a cell which can be developed to any tissue. There are two basic characteristics: self-renewal making a self by repeated division, and multipotency differentiating into cells having a specific function according to an environment.

The "mesenchymal stem cell" is a type of undifferentiated adult stem cells isolated from a human or mammalian tissue, and may be derived from various tissues. Among adult stem cells, a hematopoietic stem cell is usually non-adherent, but the mesenchymal stem cell is usually adherent. Particularly, the mesenchymal stem cell may be a umbilical cord-derived mesenchymal stem cell, a umbilical cord blood-derived mesenchymal stem cell, a bone marrow-derived mesenchymal stem cell, a adipose-derived mesenchymal stem cell, a muscle-derived mesenchymal stem cell, a nerve-derived mesenchymal stem cell, a skin-derived mesenchymal stem cell, an amnion-derived mesenchymal stem cell, or a placenta-derived mesenchymal stem cell, and preferably, a umbilical cord blood-derived mesenchymal stem cell. A technique of isolating a stem cell from each tissue is already known in the art.

The "conditioned medium of stem cells" is a material containing components included in a medium obtained by culturing stem cells, and any type of stem cells may be used to prepare the conditioned medium without limitation. For example, the stem cells for preparing the conditioned medium may be embryonic stem cells, or adult stem cells. Moreover, the adult stem cells may be derived from every tissue. In an exemplary embodiment of the present invention, a conditioned medium is prepared using umbilical cord blood-derived adult stem cells.

The "differentiation" means a phenomenon of specializing structures or functions of cells when divided, proliferated and grown, that is, a change in morphology or function of cells or tissues of an organism to perform own works. Generally, it is the phenomenon of dividing a relatively simple system into at least two qualitatively different partial systems.

The term "proliferation" or "growth" of cells refers to amplification of homogeneous cells by division, that is, an increase in the number of cells generally in a multicellular organism. When the number of cells reaches a certain level by proliferation (amplification), a character is generally changed (differentiated) and controlled at the same time.

The "medium" means a mixture for growing and proliferating cells out of an organism including essential factors used in growth and proliferation of cells, for example, saccharides, amino acids, various types of nutrients, serum, growth factors and minerals. Particularly, the medium of the present invention is a medium for growth and proliferation of stem cells.

The "basal medium" is a mixture including essential saccharides, amino acids, water, etc., required for cells to live, and a mixture excluding nutritive substances and various types of growth factors. The basal medium of the present invention may use an artificially synthesized medium or a commercially-prepared medium. The commercially-prepared medium may be, but is not limited to, for example, a Dulbecco's modified Eagle's medium (DMEM), an endothelial differentiation medium (EDM), a minimal essential medium (MEM), a basal medium eagle (BME), RPMI 1640, F-10, F-12, an α-minimal essential medium (a-MEM), a Glasgow's minimal essential medium (G-MEM), or an Iscove's Modified Dulbecco's Medium.

The "treatment" means an approach to obtain beneficial or exemplary clinical result. For the object of the present invention, the beneficial or exemplary result unlimitedly includes palliation of symptoms, reduction of a disease range, stabilization of a disease condition (that is, not deteriorated), delay of progression of a disease or reduction of a disease progression rate, improvement or temporal palliation and reduction of a disease condition (partially or totally), detectable or undetected. The "treatment" denotes all of therapeutic treatments, and preventive or precautionary methods. The treatments include treatments required for preventive disability and already-happening disability. The "palliation/palliating" of a disease means that a range of the disease condition and/or non-exemplary clinical symptoms are reduced and/or a time course of progression of the disease is delayed or extended.

The "effective amount" is a suitable amount affecting a beneficial or exemplary clinical or biochemical result. The effective amount may be administered once or more. The effective amount is a suitable amount to temporally palliate, improve, stabilize, restore, or delay the progression of a disease. In the present invention, the effective amount is an amount suitable for reducing or delaying the progression of hair loss, or stimulating hair growth. If given animals endure the administration of the composition, or the composition is appropriate to be administered to the animals, it means that the composition is "pharmaceutically or physiologically available." When the amount of the composition to be administered is physiologically important, it can be said that a preparation is administered at a "therapeutically effective amount." When the existence of the preparation brings about a physiologically detectable change in a given patient, the preparation is physiologically significant.

The term "approximately" indicates a reference amount, level, value, number, frequency, percentage, dimension, size, quantity, weight or length changed by 30, 25, 20, 15, 10, 9, 8, 7, 6, 5, 4, 3, 2, or 1%.

Throughout the specification, unless the context requires otherwise, it should be understood that the "include" and/or "including" and "comprise" and/or "comprising" specify the presence of steps or elements, or groups thereof, but do not preclude the presence or addition of one or more other steps or elements, or groups thereof.

Hereinafter, the present invention will be described in detail.

### [Stem cells]

The present invention relates to a use of stem cells having a small sized diameter to prevent hair loss and stimulate hair growth.

Stem cells are cells having a self-renewal capability, and a capability of differentiating into at least two cells. Depending on source origins, embryonic stem cells or adult stem cells may be used, and in the present invention, preferably, adult stem cells derived from various tissues originated from various sources, for example, stem cells derived from a tissue such as adipose, uterus, bone marrow, muscle, placenta, umbilical cord blood or epidermis, may be used. More particularly, the stem cells are mesenchymal stem cells (MSCs). The mesenchymal stem cells are generally stroma helping hematogenesis, and have capability of differentiating into various mesodermal cells including bone, cartilage, adipose and muscle cells, and are easily proliferated while maintaining an undifferentiated state. In one exemplary embodiment of the present invention, adipose-, bone marrow- and umbilical cord blood-derived mesenchymal stem cells (MSCs) are used.

Most preferably, umbilical cord blood-derived mesenchymal stem cells are used.

Umbilical cord blood is blood generated from the umbilical cord after birth, containing large amounts of hematopoietic stem cells and endothelial progenitor cells forming white blood cells and red blood cells, and platelets, and also containing mesenchymal stem cells forming cartilages and bones, muscles, and nerves, and thus is highly valuable in a medical field. The umbilical cord blood has a higher concentration of hematopoietic stem cells than those in the bone marrow or peripheral blood, and less matured. Accordingly, compared to hematopoietic stem cells found in the bone marrow, the hematopoietic stem cells in the umbilical cord blood exhibit more excellent proliferation, self-renewal and differentiation capability. In addition, umbilical cord blood can be obtained from discarded umbilical cord through simple medical procedures, and since the umbilical cord includes much more hematopoietic stem cells and stem cells than the given amount, in one aspect of the present invention, mesenchymal stem cells isolated from human umbilical cord blood are used.

Particularly, the umbilical cord blood-derived mesenchymal stem cells (i) can mostly avoid immunorejection unlike other tissue-derived stem cells, when used for a cell therapy product, (ii) can be taken from placenta and the umbilical cord which are generally discarded, and thus a donor does not have any pain when the stem cells are yielded, and (iii) when applied, can be directly administered to a lesion. Here, when the stem cells are actually transplanted to a corresponding lesion, a paracrine effect is activated, secretome factors (proteins, cytokines) able to treat, regenerate or restore a lesion are secreted, and thus the lesion is cured. Meanwhile, the stem cells according to the present invention may be proliferated and cultured by methods known in the art.

A suitable medium may be any one developed to culture animal cells, particularly, mammalian cells, or any one which can be prepared *in vitro* with suitable components required for the growth of animal cells, for example, assimilable carbon, nitrogen and/or trace nutrients.

The medium may be any basal medium suitable for the growth of animal cells, and as an unlimited example, a minimal essential medium (MEM), a Dulbecco modified Eagle medium (DMEM), a Roswell Park Memorial Institute medium (RPMI), or a keratinocyte-serum free medium (K-SFM) may be generally used as a basal medium used for culturing cells. Other than this, any medium used in the art may be used without limitation. Preferably, the medium may be selected from the group consisting of an α-MEM medium (GIBCO), a K-SFM medium, a DMEM medium (Welgene), an MCDB 131 medium (Welgene), an IMEM medium (GIBCO), a DMEM/F12 medium, a PCM medium, an M199/F12 (mixture) (GIBCO), and an MSC expansion medium (Chemicon).

Assimilable sources of carbon, nitrogen and trace nutrients, and as an unlimited example, a serum source, a growth factor, an amino acid, an antibiotic, vitamin, a reductase, and/or a saccharide source may be added to such a basal medium. However, it is apparent that the most suitable mediums for various tissue-derived stem cells can be selected or combined, and the stem cells can be suitably cultured by those of ordinary skill in the art. In one exemplary embodiment, an α-MEM medium or a K-SFM medium is used.

It is also apparent that the stem cells can be cultured by regulating conditions such as a suitable culture environments, time, and temperature based on conventional knowledge in the art by those of ordinary skill in the art.

In one exemplary embodiment, mesenchymal stem cells were cultured and proliferated to have a cell confluence of approximately 80 to 90%, preferably, approximately 90%, in an α-MEM medium, washed with, for example, PBS, and then further cultured in a K-SFM medium for approximately 20 to 25 hours, preferably 24 hours.

The term "confluence (%)" is conventionally used in the art to express a cell density (degree of saturation) per area, and a unit usually used in experiments by those of ordinary skill in the art to relatively express the number of cells (cell density) per unit area in cell culture. The present invention also includes a method of preparing the small-sized stem cells having a diameter of 8 µm or less and a conditioned medium thereof.

Meanwhile, the method may further include an operation of treating the stem cells cultured in a medium according to the present invention with trypsine. When the cultured stem cells are treated with trypsine, single cell-type stem cells may be obtained, and here, the trypsine is treated to inhibit aggregation between cells and have single cell-type stem cells. The trypsine may be replaced with a material capable of inhibiting the formation of the aggregation between cells.

The culture of stem cells may be performed in a conventionally-known container. For example, the stem cells may be cultured using a three-dimensional bioreactor or spinner, or in a general adhesive container.

### [Function of stimulating hair growth]

The present invention relates to a use of the particularly excellent function of the small-sized stem cells having a diameter of 10 µm or less, most preferably, 8 µm or less, to prevent hair loss and stimulate hair growth.

The function of the small-sized stem cells having a diameter of 8 µm or less to prevent hair loss and stimulate hair growth is caused by normalization of a hair cycle and increases in number and thickness of hair follicles by stimulating activity of hair follicle stem cells to reduce time for converting a telogen phase into an anagen phase in the hair cycle and increase the generation of dermal papilla cells.

Human hair goes through a process in which a cycle of an anagen phase, a catagen phase and a telogen phase is repeated, and falling and regenerating hair are performed, and the hair cycle is operated by regulating hormones or various growth factors. Hair is buried in the skin and covered with epidermis and dermis, which is called a hair follicle. There is a dermal papilla, which is a tissue controlling hair, on the hair follicle, and hair mother cells forming hair right on the dermal papilla to produce new hair and push the hair upward while divided. The dermal papilla cells have a cycle consisting of an anagen phase in which the growth of the cells is active, a catagen phase in which degeneration starts, and a telogen phase. When signals are received from adjacent cells after the telogen phase, the cells reenter to the anagen phase, and the renewal of the cells is performed, and therefore new hair is produced.

Hair follicle is a skin organ only in a mammal, and generated from a prenatal phase by interaction between epidermis and mesenchyme.

The generation of hair follicle in the prenatal phase starts in response to a signal of the dermis, and thus the epidermis becomes thick and is formed in a plate. A signal of the epidermis generated from the thick epidermal plate induces aggregation of mesenchyme-derived dermal cells, and a signal of the dermis is generated again from the formed aggregate. This signal stimulates proliferation of epidermal cells, and induces penetration of the epidermal cells into the dermis to surround the aggregate, and thus a dermal papilla is formed. Accordingly, the first hair follicle structure is formed, and then proliferation and differentiation of epidermal cells occur and lead to development to a mature hair follicle producing hair. In the mature hair follicle, specialized differentiation of hair matrix cells occurs by interaction between the hair matrix cells and dermal papilla cells through a basal membrane of the hair follicle, and thus the hair is produced and grown. In addition, the interaction leads to the cycle of the hair follicle, maintains an organ, and determines biological characteristics such as the thickness and morphology of the hair.

Two important factors regulating the biological characteristics in the hair follicle are an outer root sheath (ORS) cell, which is hair follicle epidermis, and a mesenchyme-derived dermal papilla (DP), and hair is grown and fallen through repetition of the hair cycle.

A stratum corneum and hair are produced and fallen through proliferation and differentiation of a skin surface and a hair follicle, respectively, and a basic cell source continuously supplementing, maintaining and regenerating them is stem cells. It is known that the hair follicle has a container of epidermal stem cells in a swelling part, which is involved in maintenance of the hair follicle, and renewal of sebaceous glands and hair shaft epidermis. In addition, there are mesenchyme-derived transit-amplifying cells in a dermal sheath (DS) under the hair follicle to maintain a dermal papilla of the hair follicle in the anagen phase, and the mesenchyme-derived transit-amplifying cells are known as a cell source which is able to replace a component of a dermis of a biosynthetic skin, that is, fibroblast, and become an object of study. Therefore, it can be seen that the hair loss can be effectively treated by the method of activating hair follicle stem cells in the telogen phase of the present invention.

Particularly, the small-sized stem cells having a diameter of 8 µm or less of the present invention considerably reduce the time for converting the telogen phase into the anagen phase in the hair cycle. That is, the regulation of the hair cycle is normalized by stimulating the activation of the hair follicle stem cells in the telogen phase.

Hereinafter, characteristics of the function of the small-sized stem cells having a diameter of 8 µm or less of the present invention to prevent hair loss and stimulate hair growth will be described.
(i) The small-sized stem cells having a diameter of 8 µm or less of the present invention activate stem cells involved in generation of dermal papilla cells and stem cells involved in extension of hair length.
   It has been reported that conventional sources enabling hair growth affect only a part of various factors required to induce hair growth. For example, in the known prior documents, the activation of hair follicle stem cells involved in hair growth is the activation of stem cells involved in the production of dermal papilla (DP) or stem cells involved in the extension of hair length.
   Contrarily, according to the present invention, as most of hair-related hair follicle stem cells are activated in addition to the two types of stem cells, the number and thickness of hair follicles are increased, and the thickness of the tissue is also increased. That is, the present invention provides a source affecting various factors at the same time, and used for combined treatment for hair loss and stimulation of hair growth.
(ii) The small-sized stem cells having a diameter of 8 µm or less of the present invention greatly reduces the time for converting the telogen phase into the anagen phase in the hair cycle.

Therefore, the small-sized stem cells having a diameter of 8 µm or less of the present invention exhibits a permanent hair growth effect by normalizing hair cycle regulation, not a temporal effect on hormones.

The conventional drugs including Minoxidil® delay the progression of hair loss or help in maintenance of current hair in the anagen phase without permanent treatment, and thus when the use of the drugs is stopped, hair loss immediately occurs.

However, the small-sized stem cells of the present invention is able to fundamentally treat hair loss based on a permanent hair growth effect since the number and thickness of hair follicles are increased and production of dermal papilla (DP) is increased through normalization of the hair cycle.

In one exemplary embodiment of the present invention, it is confirmed that small-sized umbilical cord blood-derived mesenchymal stem cells have the shortest time for converting into the anagen phase. Moreoever, compared to Minoxidil® and PRP which is widely known as a medicine for hair growth, small-sized umbilical cord blood-derived mesenchymal stem cells more considerably increase the number and thickness of hair follicles, that is, they shows the highest effect on hair growth

In addition, it is confirmed that the small-sized stem cells having a diameter of 8 µm or less of the present invention or a conditioned medium thereof exhibits a high effect of stimulating hair growth by activating hair follicle stem cells, compared to conventional heterogeneous stem cells in which various sizes of stem cells are mixed.

As conventionally known, when mesenchymal stem cells are cultured, the stem cells having various size distributions are mixed. However, in the present invention, when only the small-sized cells having a diameter of 8 µm or less of the present invention are extracted to culture, an effect of stimulating hair growth is considerably exhibited.

That is, for the effect of preventing hair loss and stimulating hair growth, the "size" of the stem cells is a very important factor, small-sized stem cells having a diameter of 8 µm or less have an excellent effect of reducing melanin, regardless of a type of tissue such as adipose, bone marrow or umbilical cord blood from which stem cells are derived, and particularly, small-sized umbilical cord blood-derived stem cells having a diameter of 8 µm or less have most excellent effect.

### [Composition and use thereof]

Therefore, in one aspect, the present invention provides a composition for use in therapy of hair loss for preventing hair loss and stimulating hair growth, comprising small-sized stem cells having a diameter of 8 µm or less, or a conditioned medium thereof as an active ingredient, a method of using the same through percutaneous administration using injection, and a non-therapeutic, cosmetic use of a cosmetic composition for preventing hair loss and stimulating hair growth, wherein the composition is comprising small-sized stem cells or a conditioned medium thereof as defined herein.

The stem cells may be contained in an effective concentration in a range not exhibiting cytotoxicity of 10 to 30%(v/v), preferably, 15 to 25%(v/v), and most preferably 20%(v/v), but the present invention is not limited thereto.

As an exemplary embodiment of the present invention, the composition for use of the present invention includes a pharmaceutical composition. As further exemplary embodiment, the present invention includes a non-therapeutic, cosmetic use of a cosmetic composition for preventing hair loss and stimulating hair growth, wherein the composition is comprising small-sized stem cells or a conditioned medium as defined herein.

### Pharmaceutical composition

The present invention provides a pharmaceutical composition for use in therapy of hair loss for preventing hair loss and stimulating hair growth, which contains small-sized stem cells having a diameter of 8 µm or less, or a conditioned medium thereof as an active ingredient.

Hair loss or alopecia is largely classified into cicatricial alopecia and non-cicatricial alopecia, and the non-cicatricial alopecia includes congenital alopecia, male pattern alopecia and alopecia areata, and in the present invention, all of the types are included, but the present invention is not limited thereto.

A pharmaceutically available carrier included in the pharmaceutical composition of the present invention is one conventionally used in preparation, which includes, but is not limited to, lactose, dextrose, sucrose, sorbitol, mannitol, starch, acacia rubber, calcium phosphate, alginate, gelatine, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil. The pharmaceutical composition of the present invention may further include a lubricant, a wetting agent, a sweetening agent, a flavoring agent, an emulsifier, a suspension, or a preservative in addition to the components described above.

A suitable dose of the pharmaceutical composition for use according to the present invention varies due to causes such as a preparation method, an administration method, a patient's age, body weight, sex, severity of a disease, diet, administration time, an administration route, an excretion rate and reaction sensitivity, and an ordinarily-skilled doctor may easily determine a effective dose on desired treatment, and prescribe the effective dose of pharmaceutical composition. Meanwhile, the dose of the pharmaceutical composition of the present invention may be, but is not limited to, 0.01 to 2000 mg/kg (body weight) per day.

The pharmaceutical composition for use according to the present invention may be orally or parenterally administered, and when parenterally administered, the pharmaceutical composition may be administered by intravenous injection, subcutaneous injection, muscular injection, abdominal injection, or percutaneous administration, and preferably parenteral administration. The administration route of the pharmaceutical composition for use according to the present invention may be determined according to a type of a disease to which the pharmaceutical composition is applied.

For example, the pharmaceutical composition for use according to the present invention is most preferably administered by local application to skin. A region to which the composition for use according to the present invention can be applied may be any region of the body requiring hair growth, in addition to a scalp. For example, the pharmaceutical composition may be used on a region in which hair is damaged due to a scar caused by injury, or a wide forehead or M-shape forehead, eyelashes or an eyebrow for a simple cosmetic effect, and to improve the condition of atrichosis.

Most preferably, the composition for use according to the present invention is administered by percutaneous administration through injection.

Here, to flow the composition through a capillary after sufficient diffusion into the dermis in the skin, that is, to prevent from shortly passing the dermis without sufficient diffusion and flowing into the body immediately, the composition may be injected while placing a hole in a syringe needle upward.

### Cosmetic composition

In still another aspect, the present invention provides a non-therapeutic cosmetic use of a cosmetic composition for preventing hair loss and stimulating hair growth, which contains small-sized stem cells having a diameter of 8 µm or less, or a conditioned medium thereof as an active ingredient.

The cosmetic composition that is used in the present invention may be prepared in any product form conventionally prepared in the art, for example, an emulsion, a cream, a face lotion, a pack, a foundation, a lotion, a fluid, or hair cosmetic, but the present invention is not limited thereto.

The composition may be prepared in a type of a shampoo, a hair rinse, a hair tonic, a hair gel, a hair lotion, a hair pack, a hair spray, a hair mousse, hair treatment, a hair color, a hair conditioner, a mixture thereof, for example, a mixture of a shampoo and a rinse, a mixture of a rinse and a treatment, or a liquid-type hair growth solution for stimulating hair growth by adding a conventional additive, and an aerosol-type composition may also be included.

When the product form of the cosmetic composition that is used in the present invention is a paste, a cream or a gel, as a carrier component, animal oil, vegetable oil, wax, paraffin, starch, tragacanth, a cellulose derivative, polyethylene glycol, silicone, bentonite, silica, talc or zinc oxide may be used. When the form of the cosmetic composition that is used in the present invention is a powder or a spray, as a carrier component, lactose, talc, silica, aluminum hydroxide, calcium silicate or polyamide powder may be used, and particularly, when the form of the cosmetic composition that is used in the present invention is a spray, a propellant such as chlorofluorohydrocarbon, propane/butane or dimethyl ether may be additionally included. When the form of the cosmetic composition that is used in the present invention is a solution or an emulsion, as a carrier component, a solvent, a solubilizer or an emulsifier, for example, water, ethanol, isopropanol, ethyl carbonate, ethyl acetate, benzyl alcohol, benzyl benzoate, propyleneglycol, 1,3-butylglycol oil, glycerol aliphatic ester, polyethylene glycol or sorbitan fatty acid ester may be used. When the form of the cosmetic composition that is used in the present invention is a suspension, as a carrier component, a liquid-phase diluent such as water, ethanol or propylene glycol, a suspension such as ethoxylated isostearyl alcohol, polyoxyethylene sorbitol ester or polyoxyethylene sorbitan ester, microcrystalline cellulose, aluminum hydroxide, bentonite, agar or tragacanth may be used. When the form of the cosmetic composition that is used in the present invention is a cleanser containing a surfactant, as a carrier component, aliphatic alcohol sulfate, aliphatic alcohol ether sulfate, sulfosuccinate monoester, isethionate, an imidazolium derivate, methyltaurate, sarcosinate, fatty acid amide ether sulfate, alkylamidobetaine, aliphatic alcohol, fatty acid glyceride, fatty acid diethanolamide, vegetable oil, a lanolin derivative or ethoxylated glycerol fatty acid ester may be used.

A component included in the cosmetic composition that is used in the present invention may include components conventionally used in the cosmetic composition, for example, conventional adjuvants including an antioxidant, a stabilizer, a solubilizer, a vitamin, a pigment and a fragrance, and a carrier, in addition to the active ingredient.

The cosmetic composition may be prepared by any conventionally used method.

The cosmetic composition for preventing hair loss and stimulating hair growth may be used by percutaneous administration such as direct application or injection to a scalp or hair.

An applied amount of a mixed extract which is an active ingredient included in the composition of the present invention may be 40 mg/kg or less, and preferably, 20 to 40 mg/kg based on an adult.

All of methods of applying the composition to the skin known in the art may be used. The cosmetic composition that is used in the present invention may be used by single or repeated application, or application alone or in combination with another cosmetic composition. In addition, the cosmetic composition having an excellent skin protecting effect that is used in the present invention may be used according to a conventional method, and the number of uses may be changed according to a skin condition or taste of a user.

Moreover, from a different viewpoint, disclosed herein is a method of treating a hair loss using small-sized stem cells having a diameter of 8 µm or less, or a conditioned medium thereof as an active ingredient, too.

In the above method, the used stem cells or composition thereof are the same as previously described.

In the method of treating a hair loss, in particular, the stem cells or composition thereof are preferably administered through percutaneous administration using injection. Here, to flow the composition through a capillary after sufficient diffusion into the dermis in the skin, that is, to prevent from shortly passing the dermis without sufficient diffusion and flowing into the body immediately., the composition may be injected while placing a hole in a syringe needle upward

As described above, the small-sized stem cells having a diameter of 8 µm or less, or a conditioned medium thereof was described based on the pharmaceutical composition for use according to the present invention and the cosmetic composition that is used in the present invention, but it is apparent to those of ordinary skill in the art that the present invention includes various types of compositions and methods of using the compositions having an effect of preventing hair loss and stimulating hair growth including small-sized stem cells having a diameter of 8 µm or less, or a conditioned medium thereof as an active ingredient.

That is, the small-sized stem cells having a diameter of 8 µm or less, or a conditioned medium thereof, that is present in the composition for use according to the present invention, or that is used in the present invention, stimulates the activation of hair follicle stem cells to shorten the time for converting a telogen phase into an anagen phase in a hair cycle and increases generation of dermal papilla cells, and thus has a very excellent effect of preventing hair loss and stimulating hair growth, and the present invention also includes various uses based on such an effect.s

### Advantageous Effects

According to the present invention, small-sized stem cells having a diameter of 8 µm or less or a conditioned medium thereof stimulates the activity of hair follicle stem cells to reduce time for converting a telogen phase into an anagen phase in a hair cycle and increase generation of dermal papilla cells, thereby exhibiting a very excellent hair loss preventing and hair growth stimulating effect, and thus it is very useful in an applicable field.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1 is a schematic diagram illustrating a method of manufacturing a fragment to effectively observe the length or the number of hair follicles when a mouse tissue is taken to observe the hair follicles;
FIG. 2 is an image showing hair growth effects of a control and hUCB-MSC groups of C3H mice, which are observed with the naked eye;
FIG. 3 is a microscope image showing the comparison of the number and length of hair follicles, and a skin thickness in the control and the hUCB-MSC groups of the skin tissues of the C3H mice;
FIG. 4 is an image of comparing hair growth effects in a C3H mouse of small-sized cells isolated from a control, and adipose-, bone marrow- and umbilical cord blood-derived mesenchymal stem cells, which are observed with the naked eye at the 5th to 7th weeks;
FIG. 5 is an image showing a combination of results of animal histological analysis for hair growth effects (at the 7th week) in the C3H mouse of small-sized cells isolated from a control, and adipose-, bone marrow- and umbilical cord blood-derived mesenchymal stem cells;
FIG. 6 is an image showing a combination of results of animal histological analysis for hair growth effects (at the 8th week) in the C3H mouse of a conditioned medium of small-sized cells isolated from a control, and adipose-, bone marrow- and umbilical cord blood-derived mesenchymal stem cells;
FIG. 7 is a graph showing a CCk-8 experiment result to confirm an effect of small-sized hUCB-MSCs on HaCaT cell proliferation according to time; and
FIG. 8 is an image showing an effect of proliferating dermal papilla (DP) cells in a control, adipose-, bone marrow- and umbilical cord blood-derived mesenchymal stem cells through live/dead staining.

### Best Mode Carrying out the Invention

Hereinafter, the present invention will be described in further detail with reference to examples. The examples are merely provided to explain the present invention, and it is apparent to those of ordinary skill in the art that it will not be construed that the scope of the present invention is limited to the examples.

The inventors isolated various sizes of stem cells derived from various tissues to observe an effect on growth of hair cells and confirm an effect of stimulating hair growth. It is based on the facts that small-sized stem cells make the senescence of the cells delayed; and the older cell become, the larger the size of the cell become and the lower a proliferation rate is .

### Materials and methods

### 1. Preparation of stem cells

### (1) Isolation of stem cells

In the present invention, human umbilical cord blood-derived mesenchymal stem cells provided from Medipost Co., Ltd. (Korean) were used. The cells can be obtained by taking umbilical cord blood and isolating mesenchymal stem cells from the umbilical cord blood and culturing the stem cells, and detail descriptions for the operations are as follows:
In the operation of taking umbilical cord blood, in normal vaginal delivery, cord blood is taken from an umbilical vein extracted while placenta still remains in uterus after birth, or in cesarean section, cord blood is taken from an umbilical vein while placenta is also extracted from uterus after birth.

In the present invention, when the cord blood is taken from the umbilical vein extracted from uterus after birth, it may be taken from the umbilical vein linking the placenta to a fetus by aseptic manipulation after birth. First, the umbilical vein is obtained, and then the cord blood is put into an umbilical cord blood pouch containing an anticoagulant using a collecting needle.

Methods of isolating and culturing mesenchymal stem cells from the taken umbilical cord blood may be any of the conventionally used methods including the method disclosed in Korean Patent No. 10-0494265 (Pittinger MF, Mackay AM, et al., Science, 284: 143-7, 1999; Lazarus HM, Haynesworth SE, et al., Bone Marrow Transplant, 16: 557-64,1995).

Monocytes were isolated from the obtained umbilical cord blood through centrifugation, washed several times to remove debris, seeded in a culture container at a suitable density, and cultured. After the cells were proliferated in a single layer, homogeneous, spindle-shaped cells proliferated in the form of colony were identified as mesenchymal stem cells using a phase microscope. In addition, when the cells were grown to be confluent, the cells were proliferated to be the number of cells as needed through sub-culture.

Meanwhile, various cells used as a control were HEK293 cells, adipocytes (ATCC, USA), Bone marrow (LONZA, USA), hDPC, and HaCat (given by Chung Ang University).

### (2) Preparation of conditioned media

Conditioned media were prepared from hUCB-MSC, BM-MSC, and Adipose-MSC. The cells in a storage state (stored in an LN2 tank) were defrosted and cultured in an incubator containing 5% CO₂ at 37°C, and proliferated in an α-MEM (GIBCO) medium to have cell confluence of approximately 90%.

Afterward, the cells were washed three times with phosphate buffered saline (PBS), incubated in a keratinocyte-serum free medium (K-SFM) to which phenol red was not added for 24 hours to obtain a conditioned medium, and the procedure described above was repeated for 3 days. In addition, the obtained conditioned medium was filtered (Top Filter System, Nunc), and then stored in a refrigerator and frozen.

### 2. Co-culture

hUCB-MSC (5000, 10,000, 15,000, 20,000 cells/top chamber) was co-cultured in a top chamber (pore size: 1 µm) along with hair-related cells, DPs and HaCat in a transwell chamber (Falcon, USA).

### 3. Isolation of small-sized cells

The cells in the storage state (stored in the LN2 tank) were defrosted and cultured in a 5% CO₂ incubator at 37°C, and small-sized stem cells were isolated and obtained using 8 and 20 µm membrane filters.

Here, cells having a diameter of 8 to 20 µm were obtained using a 20 µm membrane filter, and then using an 8 µm membrane filter.

In addition, the stem cells were tested by dividing them into three groups including a group 1 of hetero stem cells in which various sizes of stem cells were mixed before isolation, a group 2 of stem cells having a diameter of 8 µm or less, and a group 3 of stem cells having a diameter of 20 µm or more.

### 4. Preparation of C3H mouse for observing hair growth

As places for an experiment, Medipost Co., Ltd (IRB Approval No: 131021-1) and the Gyeonggi Biocenter (IACUC Project No: IACUC2014-4-10) were used, and a C3H mouse was purchased from Saeronbio Inc., and prepared in the Jackson Lab.

Particularly, the C3H mouse (Jackson Lab, Japan) used in the experiments of the present invention is a mouse model used as a hair growth effectiveness model in which a telogen phase is initiated when hair is removed, and unlike other kinds, it takes very long time to be converted into an anagen phase. That is, unlike other kinds of mice, the C3H mouse is an excellent animal model to confirm a hair growth effect without treatment of a telogen phase-inducing drug, due to a very long period of the telogen phase (Journal of Investigative Dermatology (2005) 124, 288-289).

A surface of the skin of the C3H mouse becomes black in an anagen phase in which hair is growing, and becomes pink in a catagen phase, and thus the time of growing hair may be detected by observing the color of the mouse skin.

The inventors obtained 7 week-old CH3 mice, and mice were adapted to an environment through one-week acclimation. In addition, the inventors intended to observe the time to be converted from the telogen phase into the anagen phase of the hair cycle by injecting the cord blood-derived mesenchymal stem cells into the mouse model. In this example, the mouse model to which PBS was injected was used as a negative control.

Meanwhile, for shaving, the mouse was anesthetized. 15.83 ml of a solution was prepared as an anesthetic by mixing 3.36 ml Rompun (Bayer Korea, 2094L, Korea) in 5ml zoletil (Bar code # 3UHC, Korea), and adding 7.47 ml saline (JW Pharmaceutical, REG# 10055, Korea), and the mouse was anesthetized with 20 µl of the solution.

In addition, after the anesthetization, the mouse was shaved using a hair trimmer. The mouse was placed on a clean paper, and hair was first shaved in an opposite direction to the hair-growing direction. After the mouse was maintained for 24 hours, and then rechecked to remove remaining hair.

### 5. Injection of stem cells of the present invention

To prevent the direct flow of the stem cells of the present invention into the body of the mouse through dermis in the skin, a hole in a syringe needle was placed to face upward, and put into the dermis of the anesthetized mouse to prevent from shortly passing the dermis without sufficient diffusion and flowing into the body immediately. Here, when the cord blood-derived mesenchymal stem cells were injected, the skin was tightly grabbed not to leak the stem cells outside, and the stem cells were completely injected by rubbing the skin to pull the needle out of the skin.

### 6. Taking of tissues of the mouse

To check the effect according to the present invention, skin tissues were taken from the mouse to which the cord blood-derived mesenchymal stem cells were injected.

A 6-well plate, forceps, operating scissors, clean paper, and a hair remover were prepared, and first, the previously grown hair of the mouse was removed. The back skin closest to the head was picked using forceps, a skin tissue was cut out using the operating scissors, and the cut tissue was pasted on the paper and cut out again in a hexagon shape. Here, the cutting was carefully performed not to damage a part of the tissue to be photographed, and performed while matching the paper to an end of the tissue to easily adhere to each other.

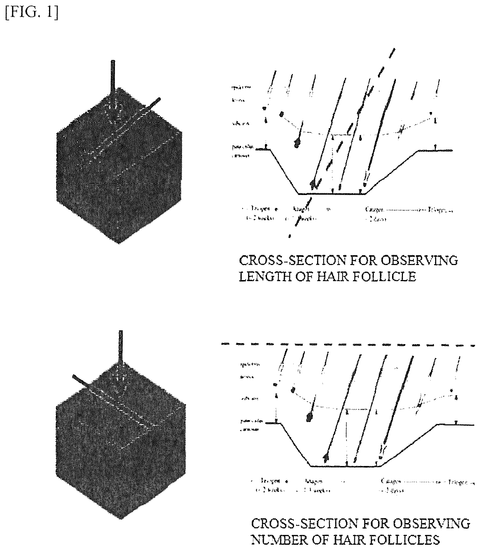

FIG. 1 is a schematic diagram showing a direction of preparing a fragment for observing the length and thickness of hair follicles (top) and a direction of preparing a fragment for observing the number and size of hair follicles (down). As tissues were taken in these directions, a lengthwise cross-section and a number-wise cross-section of the hair follicles can be observed.

### 7. H&E Staining

The obtained tissue was stored at -80°C, washed with PBS for 15 minutes for observation, and washed again three times with PBS containing 4% sucrose for 15 minutes. Afterward, the resulting product was reacted overnight with PBS containing 30% sucrose at 4°C. On the next day, the resulting product was washed again three times with an aqueous washing product in which 30% sucrose was dissolved for 15 minutes, and the skin tissue was cut to a thickness of 10 µm.

The prepared tissue was stained with Harris hematoxyline for 30 seconds at 25°C, and washed with water for 10 minutes. In addition, the resulting tissue was stained with eosin for 1 minute at 25°C, and washed twice with 95% alcohol for 10 seconds at 25°C. Subsequently, the tissue was washed twice with 100% alcohol for 10 seconds, reacted with xylene for 2 minutes at 25°C, and observed using a microscope (Nikon, ECLIPS E600W, Japan). The morphology of the hair follicle was able to be observed through the H&E staining, and a hair growth cycle was confirmed.

### 8. Immunohistochemistry

A sample was fixed by a reaction with 4% paraformaldehyde (PFA, Sigma, USA) for 15 minutes at 25°C. After 15 minutes, the sample was washed with cold PBS, 0.3% Triton X-100 (Sigma, USA) was added using PBS and reacted with the sample for 10 minutes at 25°C so that immune antibodies were easily penetrated into the sample.

Then, the sample was washed three times for 5 minutes, and blocked in PBS containing 1% BSA for 1 hour at 25°C. Afterward, primary antibodies of rabbit Ki-67 (1:250, Abcam, Cambridge, UK) and mouse PCNA (1:250, Abcam, Cambridge, UK) were reacted with the sample overnight at 4°C. In addition, secondary antibodies of rabbit Cy3 (1:400, Jackson Laboratory, California, USA) and mouse Alexa488 (1:400, Jackson Laboratory, California, USA) were added to perform an additional reaction for 1 hour and 30 minutes at 25°C.

Subsequently, the secondary antibodies were discarded and washed three times with PBS in a dark place not to be exposed to light. A nucleus of the sample was stained with 1 µg/ml of DAPI (1:1000; Sigma-Aldrich, St. Louis, MO, USA) for 5 minutes at 25°C, and mounted on each of a fluorescent microscope (Nikon) and a confocal microscope (ZEISS, LSM700/German, Nikon Eclipse TE2000-U/Japan) for observation.

### 9. CCK8 analysis

To detect cell proliferation, 400 µl of a culture medium and 40 µl of a reagent of a cell counting kit (CCK-8, Dojindo, USA) were treated for 1 hour under 5% CO₂ at 37°C, and the culture medium was transferred to a 96-well plate to detect an optical density (OD) at 450 nm using a spectrophotometer.

### 10. Quantitative analysis using live/dead staining

Live/dead staining is a method for staining live cells and dead cells using two colors of fluorescent probes. Dead cells are shown red at a channel wavelength of 565 to 605 nm due to ethidium homodimer (EthD), and live cells are shown green at a channel wavelength of 440 to 480 nm due to calcein.

A supernatant of the conditioned medium was removed, and the pellets were washed with PBS once or twice, and then stained with a mixture in which 2 mM of a reagent of a Live/Dead Viability/Cytotoxicity kit (Invitrogen), 20 µl of EthD-1, and 5 µl of 4 mM Calcein AM were mixed in 10 ml of PBS for 30 minutes at room temperature.

The staining was used to observe live cells or dead cells through an Incucyte™ FLR (Essen Bioscience Inc. USA, ZEISS, LSM 510 META) fluorescent image.

### Example 1: Effect of human umbilical cord blood-derived mesenchymal stem cell (hUCB-MSC) on hair growth

### 1-1. Observation of hair growth with the naked eye

A telogen phase was induced in a C3H mouse, and PBS was injected into a half of the back of the mouse as a negative control, and a hUCB-MSC 3 group and a HEK293 cell group were injected at 1x10⁶ cells/100 µl and 5x10⁵ cells/100 µl, respectively, once into one point of the other half of the back of the mouse as a test group.

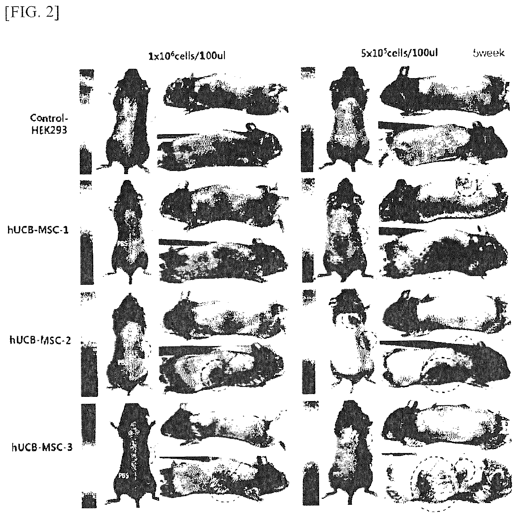

Afterward, hair growth of the mouse was observed for 5 weeks, and the result is shown in FIG. 2.

As seen from FIG. 2, only in the hUCB-MSC group, the C3H mouse showed hair growth by converting a hair cycle from the telogen phase into the anagen phase. Here, hair was longer and grown in a wider range when the cells were injected at 5x10⁵ cells, compared to when injected at 1x10^6 cells, and the group 2 of stem cells having a diameter of 8 µm or less. That is, when the hUCB-MSCs having a diameter of 8 µm or less were injected at 5x10⁵ cells/100 µl, the most excellent hair growth effect was exhibited.

### 1-2. Observation of hair follicle

Next, the inventors tried to observe the effect of the hUCB-MSC on hair growth, and the hair follicle involved in the hair growth in a C3H mouse tissue state.

To this end, a tissue fragment was manufactured by the above-described method, and stained through H&E staining to observe the number and length of hair follicles, and the thickness of a skin tissue.

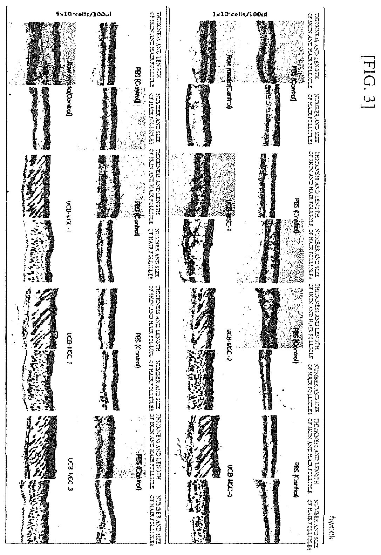

As a result, as shown in FIG. 3, in the group to which the hUCB-MSCs were injected according to the present invention, a considerably high number of hair follicles was observed, the length of the hair follicle was long, and the thickness of the skin tissue was thick twice or more. Here, when 5x10⁵ cells of the hUCB-MSCs having a diameter of 8 µm or less were injected, the largest number and the highest length of the hair follicles and the largest thickness of the skin were obtained.

### 1-3. Observation of hair growth effect of conditioned medium

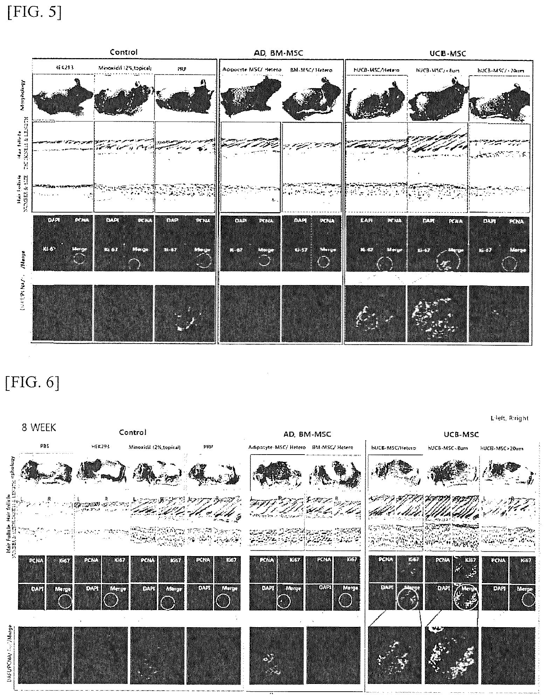

Conditioned media prepared by the method as described above (Preparation Example 1-(2)) were administered to mice by the same method, and hair growth on the mice after 8 weeks was observed, and the result is shown in FIG. 6.

As seen from FIG. 6 showing mouse and hair follicle images for hUCB-MSCs, when the conditioned medium of hUCB-MSCs having a diameter of 8 µm or less was administered, an excellent hair growth effect was shown, and particularly, compared to a conditioned medium of hUCB-MSCs having a diameter of 20 µm or more, it also can be seen from the hair follicle image that there were considerable differences in the number and length of hair follicles. According to the result, it can be confirmed that the stem cells having a diameter of 8 µm or less and a conditioned medium thereof had the most excellent hair growth effect.

### Example 2. Hair growth effect of stem cells according to various origin tissues

### 2-1. Observation of hair growth with the naked eye

The inventors compared hair growth effects of adipose-derived mesenchymal stem cells (Adipose-MSC, AD-MSCs), bone marrow-derived mesenchymal stem cells (Bone marrow-MSC, BM-MSCs) and umbilical cord blood-derived mesenchymal stem cells (hUCB-MSCs) to examine the influence of origin tissues of the stem cells on hair growth.

To this end, after the C3H mouse was induced in a telogen phase, AD-MSCs, BM-MSCs, and hUCB-MSCs were injected into one point once at an amount of 5x10^5 cells, and hair growth was observed at the 5th, 6th and 7th weeks.

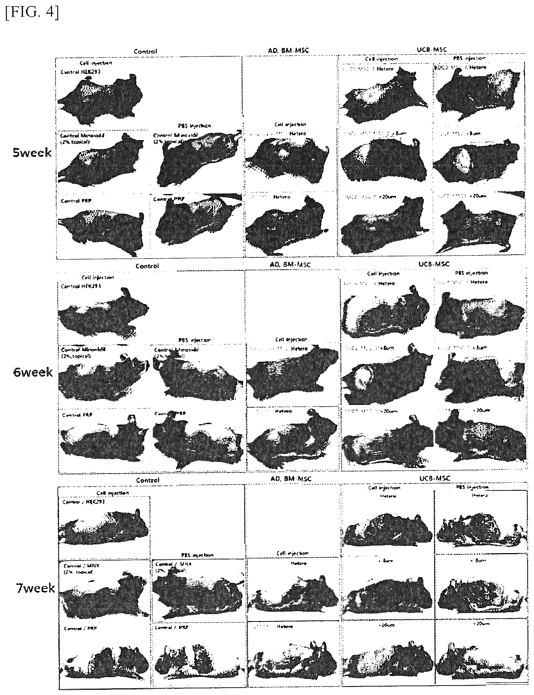

The result is shown in FIG. 4.

As shown in FIG. 4, the C3H mouse to which hUCB-MSCs were injected had the most excellent hair growth effect. A hair growth phenomenon was observed first at the 5th week, and a phenomenon in which hair was grown in 90% or more of the total area of the mouse at at least the 7th week in the group to which UCB-MSCs were injected was observed.

In addition, in comparison with a Minoxidil®group used as a positive control and a PRP group, when hUCB-MSCs were injected, a much higher hair growth effect was observed.

### 2-2. Observation of hair follicle

In addition, at the 8th week after additional cell transplantation, a hair follicle in the C3H mouse skin tissue was observed.

As a result of analyzing the number and length of the hair follicles, and the thickness of the skin tissue through H&E staining, as shown in FIG. 5, compared with AD-MSC and BM-MSC, when hUCB-MSC was used, the largest number and the highest length of hair follicles were observed, and the thickness of skin tissue was thicker twice or more.

### 2-3. conditioned medium of stem cells derived from other tissues

The conditioned media prepared by the method as described above (Preparation Example 1-(2)) were administrated into a mouse by the same method as described above to observe hair growth at the 8th week, and the result is shown in FIG. 6.

As seen from the image of FIG. 6, compared with an AD-MSC conditioned medium and a BM-MSC conditioned medium, when the hUCB-MSC conditioned medium was used, the largest number and the highest length of hair follicles were observed, and the thickness of skin tissue was larger.

### Example 3: Observation of generation of dermal papilla cells

It is already known that hair growth is deeply related to cell proliferation of a dermal papilla (DP) part. Based on the fact, the inventors intended to confirm the cell proliferation of the DP part using PCNA and Ki67 antibodies.

As a result, as shown in FIG. 5, among adult stem cells derived from various sources, when hUCB-MSC was injected, a considerable cell proliferation effect was observed in the DP part.

In addition, as shown in FIG. 6, among conditioned media s of adult stem cells derived from various sources, when hUCB-MSC was injected, a considerable cell proliferation effect was observed in the DP part.

According to the above result, among mesenchymal stem cells derived from various tissues, it was seen that umbilical cord blood-derived mesenchymal stem cells and a conditioned medium thereof are the source showing the highest hair growth effect.

### Comparative Example 1: Hair growth effect of small-sized hUCB-MSCs

### (1) In vitro experiment

To compare hair growth effects of stem cells according to a size, small-sized hUCB-MSCs having a diameter of 8 µm or less, large-sized hUCB-MSCs having a diameter of 20 µm or more, and hetero cells in which various sizes of cells were mixed were used to analyze. AD-MSCs and BM-MSCs were prepared in a hetero cell state. Minoxidil® and PRP were used as positive controls, and HEK293 cells and raw media were used as negative controls.

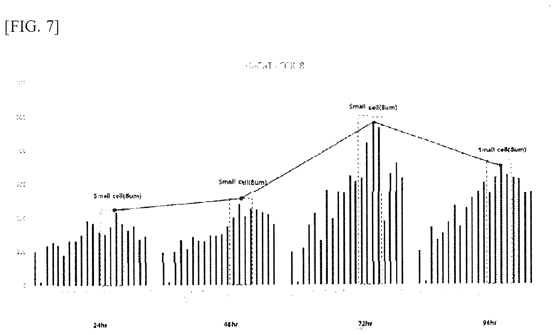

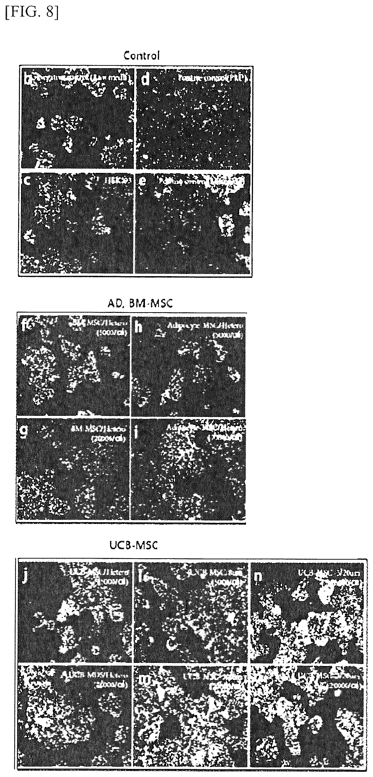

In addition, to observe the effect on proliferation of DP cells and HaCaT cells, the cells were treated in an upper chamber in vitro using a concurrent culture system. The cells were treated for total 96 hours to observe, and the cell proliferation in CCK-8 was detected through cell counting every 24 hours. The results are shown in FIGS. 7 and 8.

It was confirmed that proliferation levels of the DP and HaCaT cells were highest at 72 hours, and hUCB-MSCs showed twice or higher cell proliferation than the hetero cells.

In addition, in terms of the difference in proliferation according to the size of hUCB-MSCs, in the small-sized hUCB-MSCs having a diameter of 8 µm or less, the proliferation of the DP cells were 4.8 times or higher than that of the control group (FIG. 7).

In addition, the stem cells seeded in the upper chamber in the concurrent culture system were cultured in various numbers from 500 to 20,000 cells, and when the number of stem cells was 15,000, the highest cell proliferation was shown (FIG. 8).

In addition, it was confirmed through the live/dead staining that the small-sized hUCB-MSCs having a diameter of 8 µm or less had the highest DP cell proliferation effect (FIG. 8).

### (2) In vivo experiment

Like the in vitro result described above in which hair growth was observed according to a cell size, results obtained by confirming the in vivo hair growth effect by injecting cells into the C3H mouse are shown in FIG. 5 and 6.

According to the result obtained through H&E staining, small-sized hUCB-MSCs having a diameter of 8 µm or less and a conditioned medium thereof exhibited excellent effects in the number and length of hair follicles, and the skin thickness. The same result was yielded by the comparison examined using PCNA and Ki67 fluorescent staining.

According to such results, it was seen that stem cells having a small diameter of 8 µm or less, particularly, small-sized umbilical cord blood-derived stem cells having a diameter of 8 µm or less and a conditioned medium thereof stimulated DP cell proliferation and growth of a hair follicle and thus hair growth, thereby exhibiting the most excellent function in prevention of hair loss and stimulation of hair growth, and compared to when heterogeneous stem cells were simply cultured without isolation by a size shown in the conventional case, a particularly outstanding effect was exhibited.

In addition, when large-sized cells having a diameter of 20 µm or more and a conditioned medium thereof was used, there was no significant effect on stimulation of hair growth, and thus it was seen that the prevention of hair loss and the stimulation of hair growth of the stem cells were greatly influenced by the size of the stem cells.

According to the present invention, small-sized stem cells having a diameter of 8 µm or less or a conditioned medium thereof stimulates the activity of hair follicle stem cells to reduce time for converting a telogen phase into an anagen phase in a hair cycle and increase generation of dermal papilla cells, thereby exhibiting a very excellent hair loss preventing and hair growth stimulating effect, and thus it is very useful in an applicable field.

## Claims

1. A composition for use in therapy of hair loss for preventing hair loss and stimulating hair growth, comprising:
small-sized stem cells having a diameter of 8 µm or less, or a conditioned medium thereof as an active ingredient.

2. The composition for use according to claim 1, wherein the small-sized stem cells having a diameter of 8 µm or less include at least one selected from the group consisting of bone marrow-, umbilical cord blood-, adipose-, blood-, liver and intestine-, skin-, gastrointestinal tract-, placenta-, nerve-, adrenal-, epithelium- and uterus-derived human adult stem cells, and embryonic stem cells.

3. The composition for use according to claim 2, wherein the small-sized stem cells having a diameter of 8 µm or less are derived from bone marrow, umbilical cord blood or adipose.

4. The composition for use according to claim 3, wherein the small-sized stem cells having a diameter of 8 µm or less are derived from the umbilical cord blood.

5. The composition for use according to claim 4, wherein the small-sized stem cells having a diameter of 8 µm or less are umbilical cord blood-derived mesenchymal stem cells.

6. The composition for use according to claim 4, wherein the umbilical cord blood is derived from human.

7. The composition for use according to claim 1, wherein the conditioned medium includes a keratinocyte-serum free medium (K-SFM).

8. The composition for use according to claim 1, wherein the small-sized stem cells having a diameter of 8 µm or less or the conditioned medium thereof activates hair follicle stem cells.

9. The composition for use according to claim 8, wherein the small-sized stem cells having a diameter of 8 µm or less or the conditioned medium thereof have the following functions:
(i) reduction of time for converting a telogen phase into an anagen phase in a hair cycle;
(ii) normalization of hair cycle regulation; and
(iii) increase in generation of dermal papilla cells.

10. The composition for use according to claim 1, wherein the composition is a pharmaceutical composition.

11. Non-therapeutic, cosmetic use of a cosmetic composition for preventing hair loss and stimulating hair growth, wherein the composition is comprising small-sized stem cells or a conditioned medium thereof as defined in any of claims 1 to 9.

12. The composition for use according to any one of claims 1 to 10 whereby administration is done through percutaneous administration using injection.

13. The composition for use according to claim 12, wherein the injection is a method of administering the composition to dermis of a target while placing a hole of a syringe needle upward.

14. The non-therapeutic cosmetic use according to claim 11, wherein the cosmetic composition is used through percutaneous administration using injection.

15. The non-therapeutic cosmetic use according to claim 14, wherein the injection is a method of administering the composition to dermis of a target while placing a hole of a syringe needle upward.

## Patentansprüche

1. Zusammensetzung zur Verwendung bei der Therapie von Haarausfall zur Verhinderung von Haarausfall und zur Stimulierung des Haarwachstums, umfassend:
kleine Stammzellen mit einem Durchmesser von 8 µm oder weniger, oder ein konditioniertes Medium davon als Wirkstoff.

2. Zusammensetzung zur Verwendung nach Anspruch 1, wobei die kleinen Stammzellen mit einem Durchmesser von 8 µm oder weniger mindestens eine aus der Gruppe bestehend aus Knochenmark-, Nabelschnurblut-, Fett-, Blut-, Leber- und Darm-, Haut-, Magen-Darm-Trakt-, Plazenta-, Nerven-, Nebennieren-, Epithel- und Uterus-abstammenden menschlichen adulten Stammzellen und embryonalen Stammzellen einschließen.

3. Zusammensetzung zur Verwendung nach Anspruch 2, wobei die kleinen Stammzellen mit einem Durchmesser von 8 µm oder weniger aus Knochenmark, Nabelschnurblut oder Fett stammen.

4. Zusammensetzung zur Verwendung nach Anspruch 3, wobei die kleinen Stammzellen mit einem Durchmesser von 8 µm oder weniger aus dem Nabelschnurblut stammen.

5. Zusammensetzung zur Verwendung nach Anspruch 4, wobei die kleinen Stammzellen mit einem Durchmesser von 8 µm oder weniger aus Nabelschnurblut abstammende mesenchymale Stammzellen sind.

6. Zusammensetzung zur Verwendung nach Anspruch 4, wobei das Nabelschnurblut vom Menschen stammt.

7. Zusammensetzung zur Verwendung nach Anspruch 1, wobei das konditionierte Medium ein keratinozytenserumfreies Medium (K-SFM) einschließt.

8. Zusammensetzung zur Verwendung nach Anspruch 1, wobei die kleinen Stammzellen mit einem Durchmesser von 8 µm oder weniger oder das konditionierte Medium davon Haarfollikel-Stammzellen aktivieren.

9. Zusammensetzung zur Verwendung nach Anspruch 8, wobei die kleinen Stammzellen mit einem Durchmesser von 8 µm oder weniger oder das konditionierte Medium davon die folgenden Funktionen haben:
(i) Verkürzung der Zeit für die Umwandlung einer Telogenphase in eine Anagenphase in einem Haarzyklus;
(ii) Normalisierung der Regulierung des Haarzyklus; und
(iii) Zunahme der Erzeugung von dermalen Papillae-Zellen.

10. Zusammensetzung zur Verwendung nach Anspruch 1, wobei die Zusammensetzung eine pharmazeutische Zusammensetzung ist.

11. Nicht-therapeutische, kosmetische Verwendung einer kosmetischen Zusammensetzung zur Verhinderung von Haarausfall und zur Stimulierung des Haarwuchses, wobei die Zusammensetzung kleine Stammzellen oder ein konditioniertes Medium davon, wie in einem der Ansprüche 1 bis 9 definiert, umfasst.

12. Zusammensetzung zur Verwendung nach irgendeinem der Ansprüche 1 bis 10, wobei die Verabreichung durch perkutane Verabreichung mittels Injektion bewirkt wird.

13. Zusammensetzung zur Verwendung nach Anspruch 12, wobei die Injektion ein Verfahren zur Verabreichung der Zusammensetzung an die Dermis eines Ziels ist, während ein Loch einer Spritzennadel nach oben platziert wird.

14. Nicht-therapeutische, kosmetische Verwendung nach Anspruch 11, wobei die kosmetische Zusammensetzung durch perkutane Verabreichung mittels Injektion verwendet wird.

15. Nicht-therapeutische, kosmetische Verwendung nach Anspruch 14, wobei die Injektion ein Verfahren zur Verabreichung der Zusammensetzung an die Dermis eines Ziels ist, während ein Loch einer Spritzennadel nach oben platziert wird.

## Revendications

1. Composition destinée à être utilisée dans le domaine de la thérapie de la chute des cheveux pour prévenir la chute des cheveux et stimuler la croissance des cheveux comprenant :
des cellules souches de faible dimension ayant un diamètre de 8 µm ou un milieu conditionné de celles-ci en tant qu'agent actif,

2. Composition destinée à être utilisée conformément à la revendication 1,
dans laquelle les cellules souches de faible dimension ayant un diamètre de 8 µm ou moins comportent au moins une cellule souche choisie dans le groupe formé par des cellules souches de la moëlle osseuse, des cellules souches sanguines du cordon ombilical, des cellules souches, des cellules souches adipeuses, des cellules souches sanguines, des cellules souches du foie et de l'intestin, des cellules souches de la peau, des cellules souches du tractus gastrointestinal, des cellules souches placentaires, des cellules souches nerveuses, des cellules souches surrénales, des cellules souches épithéliales et des cellules souches de l'utérus dérivées de l'adulte humain et des cellules souches embryonnaires.

3. Composition destinée à être utilisée conformément à la revendication 2,
dans laquelle les cellules souches de faible dimension ayant un diamètre de 8 µm ou moins sont dérivées de la moëlle osseuse, du sang du cordon ombilical ou des glandes surrénales.

4. Composition destinée à être utilisée conformément à la revendication 3,
dans laquelle les cellules souches de faible dimension ayant un diamètre de 8 µm ou moins sont dérivées du sang du cordon ombilical.

5. Composition destinée à être utilisée conformément à la revendication 4,
dans laquelle les cellules souches de faible dimension ayant un diamètre de 8 µm ou moins sont des cellules souches mésenchymateuses dérivées du sang du cordon ombilical.

6. Composition destinée à être utilisée conformément à la revendication 4,
dans laquelle le sang du cordon ombilical est dérivé d'un humain.

7. Composition destinée à être utilisée conformément à la revendication 1,
dans laquelle le milieu conditionné est un milieu exempt de sérum de kératinocytes (K-SFM).

8. Composition destinée à être utilisée conformément à la revendication 1,
dans laquelle les cellules souches de faible dimension ayant un diamètre de 8 µm ou moins ou le milieu conditionné de celles-ci active(nt) les cellules souches du follicule pileux.

9. Composition destinée à être utilisée conformément à la revendication 8,
dans laquelle les celles souches de faible dimension ayant un diamètre de 8 µm ou moins ou le milieu conditionné de celles-ci a(ont) les fonctions suivantes :
(i) réduction de la durée de transformation d'une phase télogène en une phase anagène dans un cycle du cheveu,
(ii) normalisation de la régulation du cycle du cheveu, et
(iii) augmentation de la génération de cellules de papille dermique.

10. Composition destinée à être utilisée conformément à la revendication 1,
constituée par une composition pharmaceutique.

11. Utilisation cosmétique non-thérapeutique, d'une composition cosmétique pour prévenir les chutes de cheveux et stimuler la croissance des cheveux, cette composition renfermant des cellules souches de faible dimension ou un milieu conditionné de celles-ci tel que défini(es) selon l'une quelconques des revendications 1 à 9.

12. Composition destinée à être utilisée conformément à l'une quelconques des revendications 1 à 10,
dont l'administration est effectuée par voix percutanée en utilisant une injection.

13. Composition destinée à être utilisée conformément à la revendication 12,
dans laquelle l'injection est un procédé d'administration de la composition à un derme cible en positionnant le trou de l'aiguille d'une seringue vers le haut.

14. Utilisation cosmétique non-thérapeutique conforme à la revendication 11,
dans laquelle la composition cosmétique est administrée par voix percutané en utilisant une injection.

15. Utilisation cosmétique non thérapeutique conforme à la revendication 14,
dans laquelle l'injection est un procédé permettant d'administrer la composition à un derme cible en positionnant le trou de l'aiguille d'une seringue vers le haut.
